(19) Europäisches Patentamt
European Patent Office
Office européen des brevets

(11) Numéro de publication: **0 328 446 B1**

(12) **FASCICULE DE BREVET EUROPEEN**

(45) Date de publication de fascicule du brevet: **20.07.94**

(51) Int. Cl.[5]: **C07D 519/04**, A61K 31/475, A61K 39/395

(21) Numéro de dépôt: **89400334.2**

(22) Date de dépôt: **07.02.89**

Le dossier contient des informations techniques présentées postérieurement au dépôt de la demande et ne figurant pas dans le présent fascicule.

# (54) Conjugués de dérivé de vinca comportant une chaine détergente en position C-3.

(30) Priorité: **08.02.88 FR 8801439**

(43) Date de publication de la demande:
**16.08.89 Bulletin 89/33**

(45) Mention de la délivrance du brevet:
**20.07.94 Bulletin 94/29**

(84) Etats contractants désignés:
**AT BE CH DE ES FR GB GR IT LI LU NL SE**

(56) Documents cités:
**EP-A- 0 041 935**
**EP-A- 0 121 388**
**EP-A- 0 124 502**
**EP-A- 0 206 667**
**FR-A- 2 592 883**
**GB-A- 2 111 055**

(73) Titulaire: **LA REGION WALLONNE**
**Square du Bastion, 1A**
**B-1050 Bruxelles(BE)**

(72) Inventeur: **Bhushana Rao, KSP Siva**
**11, Rue Kwakenbienne**
**B-1331 Rosières(BE)**
Inventeur: **Dejonghe, Jean-Paul**
**94, rue Ch. Jaumotte**
**B-1350 Wawre(BE)**
Inventeur: **Collard, Marie-Paule**
**18, Rue Evenepoel**
**B-1040 Bruxelles(BE)**
Inventeur: **Trouet, André**
**29, Predikherenberg**
**B-3009 Winksele-Herent(BE)**

(74) Mandataire: **Warcoin, Jacques et al**
**Cabinet Régimbeau**
**26, avenue Kléber**
**F-75116 Paris (FR)**

EP 0 328 446 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des conjugués médicament-transporteur qui utilisent comme médicaments des alcaloïdes vinca pour le traitement du cancer. Ces drogues ont la propriété de bloquer la multiplication de cellules tumorales, mais également celle de cellules normales ayant une grande fréquence de division, comme c'est le cas des cellules hématopoiétiques et des cellules épithéliales de l'intestin. En outre, ces médicaments peuvent avoir un effet toxique pour d'autres tissus et sont respectivement cardio- et neurotoxiques.

Les dérivés d'alcaloïdes de vinca les plus utilisés actuellement sont la vinblastine et la vincristine.

La vinblastine et la vincristine sont deux dérivés dimères extraits des feuilles de Catharanthus Roseus. La vindésine est un dimère hémisynthétique obtenu à partir de la vinblastine. Ces trois dérivés sont les plus utilisés à l'heure actuelle en clinique humaine dans le traitement des divers types de maladies cancéreuses. Cette utilisation se justifie par leur très nette activité contre des tumeurs humaines, telles que la maladie de Hodgkin, les tumeurs du sein et du poumon pour la vinblastine, les lymphosarcomes, la leucémie lymphoblastique, les transformations aiguës des leucémies lymphoblastiques chroniques pour la vincristine et la vindésine. L'utilisation de ces substances est cependant limitée par leur toxicité neurologique et hématologique. Depuis plusieurs années un programme de recherche a été entrepris, qui visait à développer des nouveaux dérivés moins toxiques de vincristine et de vinblastine. De nombreux dérivés d'alcaloïdes vinca ont été synthétisés dans le but d'obtenir des activités pharmacologiques antitumorales plus intéressantes. Selon l'invention, des méthodes sont développées afin de coupler des dérivés d'alcaloïdes de vinca à des transporteurs macromoléculaires dans le but, selon l'approche conceptuelle du pilotage de médicaments, de permettre d'augmenter l'activité thérapeutique tout en réduisant la toxicité inhérente à ces médicaments. Par exemple, un transporteur, qui est proposé selon l'invention, est l'albumine humaine galactosylée qui est reconnue sélectivement par les hépatocytes et les cellules de l'hépatome humain. Des techniques sont au point afin de produire cette néoglycoprotéine dans des conditions aseptiques et apyrogènes permettant son administration à l'homme. Enfin, des hybridomes ont été créés qui sécrètent des anticorps monoclonaux ayant une haute affinité et une bonne sélectivité pour les cellules de l'hépatome humain. La conjugaison à des anticorps monoclonaux a également été réalisée.

Plus précisément, la présente invention se rapporte à de nouveaux conjugués de dérivés d'alcaloïdes de vinca du type indole-dihydroindole avec des agents de pilotage.

La spécificité de l'approche du probleme de la chimiothérapie des cancers selon l'invention repose sur le concept de pilotage des médicaments qui vise, en fait, à augmenter la sélectivité des agents antitumoraux en les couplant à des transporteurs reconnus le plus spécifiquement possible par les cellules que l'on désire toucher. Outre une activité plus grande résultant du fait que la quasi totalité du médicament administré devrait atteindre sa cible, cette approche vise à réduire considérablement la toxicité des agents antitumoraux. Cette approche est basée sur le type de lien chimique que l'on intercale entre le médicament et son transporteur. En effet, les liens, selon l'invention, sont covalents, ce qui permet d'éviter la dissociation de conjugués non covalents par simple effet de dilution ; ils sont également sélectionnés en fonction de leur stabilité dans les liquides biologiques extracellulaires et de leur réversibilité en présence d'enzymes lysosomiaux. De préférence, les conjugués médicament-transporteur qui sont développés doivent être, d'une part, reconnus par des sites spécifiques présents à la surface des cellules tumorales, mais ils doivent, d'autre part, être endocytés et rejoindre les lysosomes.

Par agent de pilotage, on entend donc désigner dans la présente demande des transporteurs macromoléculaires spécifiques, pilotant sélectivement la substance active vers des cellules cibles à traiter. Il s'agit en général de protéines telles que AHgal ou un facteur de croissance tel que l'EGF ou des fragments de protéine, notamment d'immunoglobulines ou fragments d'immunoglobuline. Par exemple, dans le cas du cancer, qui est ici concerné, des anticorps monoclonaux dirigés contre des antigènes associés aux tumeurs sont particulièrement adaptés.

Comme il a été évoqué précédemment, les systèmes de pilotage ou conjugués, impliqués par la présente invention, requièrent la présence outre de l'agent de pilotage et de la substance active d'un lien chimique covalent entre ces deux composés. Il peut s'agir d'une liaison entre une fonction chimique terminale de la substance active et une fonction chimique complémentaire de l'agent de pilotage par exemple COOH et $NH_2$. Il peut également s'agir d'un groupement chimique ou bras assurant la liaison entre les deux composés.

On trouvera la description des caractéristiques que doivent présenter les différents éléments impliqués dans le concept de pilotage dans la demande de brevet FR-A-2 584 293 (FR-85 10 234) déposée par la Demanderesse.

Les dérivés d'alcaloïdes de vinca ont fait l'objet de nombreuses recherches, études et brevets, en vue de la préparation de substances plus actives, plus sélectives et moins toxiques comme agent anti-tumoral, sous forme de conjugués.

Le couplage de dimères indole-dihydroindoles à une protéine a été envisagé jusqu'à présent essentiellement selon deux voies :

1) Couplage en position C-4

a) par l'intermédiaire d'un groupe ester dérivé du groupe hydroxyl du carbone4 du squelette de d'alcaloïde de vinca (demande de brevet européen EP-124 502). Ils s'agissait alors de coupler la vindésine par un bras hémisuccinate à une immunoglobuline.

b) par l'intermédiaire d'un dérivé organique bifonctionnel du type maléoylaminoacide, notamment Ala-Mal dans la demande de brevet luxembourgeois n° 86 212 particulièrement.

On trouve cependant d'autres couplages en C-4 selon es deux types dans les demandes de brevet EP 121 388 et EP 123 441.

2) Couplage en position C-3

a) la vindésine a été couplée directement à une immunoglobuline par l'intermédiaire d'un dérivé azide dans la demande de brevet européen EP 56 322.

b) des demandes EP 206 666 et EP 206 667 proposent également des couplages d'hydrazide de vinblastine liés par un bras du type hémisuccinate à une immunoglobuline.

La présente invention se propose d'obtenir de nouveaux conjugués de dérivés d'alcaloïdes de vinca à partir des nouveaux dérivés décrits dans les demandes de brevet FR-A-2 592 883 (FR 86 00 364) et FR-A-2 608 158 (FR 86 17 412) déposées par la Demanderesse.

La présente invention a en effet pour objet des conjugués de dérivés de vinca (indole-dihydroindole)-comportant en position C-3 une chaîne détergente d'au moins 7 atomes de carbone aliphatique liée par un lien covalent à un transporteur macromoléculaire de nature polypeptidique.

Plus particulièrement, la présente invention a pour objet des conjugués de dérivé de vinca (indoledihydroindole)-alcaloïde comportant en position C-3 une chaîne détergente d'au moins 7 atomes de carbone aliphatique, chaîne substituée à son extrémité par un radical COOH couplé à une fonction amino libre d'un transporteur macromoléculaire de nature polypeptidique par l'intermédiaire d'une liaison amide (-CONH-), caractérisés en ce qu'ils répondent à la formule I

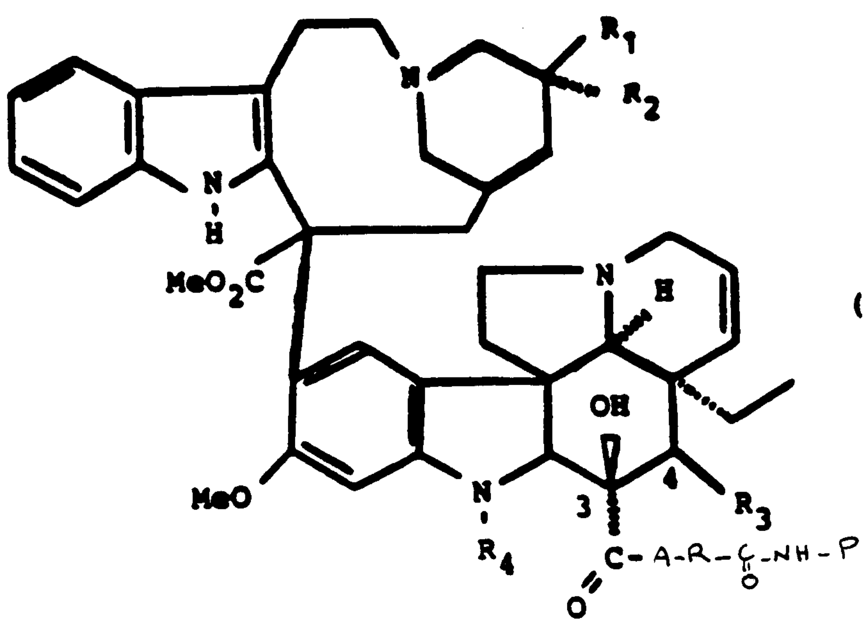

(I)

dans laquelle :

. ($R_1$, $R_2$) représentent (-Et, -OH) ou (-H, Et)

. $R_4$ représente -H, -Me ou -CHO,

. $R_3$ représente -OH ou

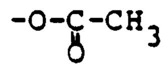

A étant -NH- ou le radical divalent d'un aminoacide entrant dans la constitution des protéines se terminant par -NH- et

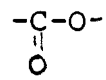

ou -NH- et

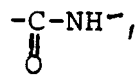

. R représente un radical hydrocarboné aliphatique en $C_7$ à $C_{20}$,

. NHP représente le reste du transporteur $NH_2P$ dans lequel $NH_2$ représente une fonction amino libre du dit transporteur.

Parmi les radicaux hydrocarbonés aliphatiques, il faut citer les radicaux :

. alkyle en $C_7$ à $C_{20}$, droits ou ramifiés, et

. alkényle en $C_7$ à $c_{20}$, droits ou ramifiés, ces radicaux étant de préférence droits.

Lorsque A est un radical divalent d'un aminoacide entrant dans la constitution des protéines, il peut être sous forme D ou sous forme L lorsqu'il comporte un atome de carbone asymétrique.

Le radical A peut provenir de Gly, Ala, Val, Leu, Ile, Phe, Ser, Thr, Lys, Lys-OH, Arg, Asp, Asp-$NH_2$, Glu, Glu-$NH_2$, Cys-SH, Met, Tyr, Trp ou His , mais de préférence Ala.

Lorsque ces radicaux présentent des fonctions autres que les fonctions de liaison, celles-ci peuvent être protégées par des groupes de protection connus dans la chimie des protéines, tels que le groupe Cbz ( carbobenzyloxy)par exemple.

On peut citer notamment les conjugués pour lesquels

. $R_3$ = -OH

et

. A = -NH- .

Parmi les conjugués de formule I, on peut également citer plus particulièrement les conjugués sous lesquels

. ($R_1$, $R_2$) = (-Et, -OH)

et

. $R_4$ = Me.

Des conjugués particulièrement intéressants sont obtenus lorsque

-A-R est choisi parmi -NH-$(CH_2)_{11}$,

-NH-$(CH_2)_{17}$ , NH-$(CH_2)_7$-CH=CH-$(CH_2)_8$.

De préférence, le transporteur macromoléculaire est choisi parmi les protéines, néoglycoprotéines et immunoglobulines IgG ou des fragments de celles-ci.

Parmi les protéines ou néoglycoprotéines, on peut citer :

. la sérum albumine de bovin ou

. l'albumine humaine galactosylée, ainsi que

. les facteurs de croissance comme l'EGF (Epidermal Growth Factor).

On peut en effet citer notamment deux types de transporteurs. Tout d'abord, des néoglycoprotéines qui exposent des résidus de galactose et qui sont reconnus par un récepteur présent à la surface des hépatocytes, mais aussi de cellules d'hépatome humain.

D'autre part, dans le cas de cellules tumorales, un autre type de transporteurs, peut être cité, en l'occurrence les anticorps monoclonaux. Cette approche tire profit de la sélectivité de la réaction antigèneanticorps et est basée sur l'hypothèse que des cellules tumorales pourraient exprimer à leur membrane plasmique des antigènes plus ou moins spécifiques. Les premiers antigènes contre lesquels on peut produire des anticorps sont les antigènes organotypiques. Ceci permet de limiter la capture des conjugués

médicaments-transporteurs à un seul type cellulaire, mais n'empêche pas l'accès du conjugué aux cellules normales exprimant les antigènes. Un second type de cible est constitué par les antigènes associés aux tumeurs. Dans cette catégorie on peut inclure les antigènes présents à la surface des cellules embryonnaires qui réapparaissent lors de la transformation maligne ou encore des antigènes présents en très petit nombre à la surface des cellules normales, mais dont le nombre augmente considérablement au cours de la cancérisation. Dans certains cas également, ce sont des antigènes d'origine virale qui apparaissent à la surface de cellules infectées et transformées. Le troisième type d'antigènes que l'on peut considérer comme cible est constitué par des antigènes spécifiques des cellules tumorales. Bien que le sujet soit encore largement controversé, il semble que des antigènes spécifiques pourraient apparaître lors de la transformation cancéreuse et être exprimés à la surface des cellules tumorales. Des anticorps dirigés contre de tels antigènes représentent les transporteurs idéaux. Un exemple d'antigène, considéré comme fonctionnellement spécifique de tumeur, est l'idiotype des immunoglobulines de surface portées par certaines cellules de leucémie B ou de lymphomes.

On peut donc citer comme transporteur l'albumine humaine galactosylée qui est reconnue sélectivement par les hépatocytes et les cellules de l'hépatome humain.

On peut également utiliser parmi des anticorps monoclonaux ayant une haute affinité et une bonne sélectivité pour les cellules de l'hépatome humain, l'anticorps CT-M-01 décrit dans la demande FR 85 10 234.

La présente invention a également pour objet un procédé de préparation de conjugués, caractérisé en ce que

1) on prépare le dérivé d'alcaloïde de vinca en C-3 à coupler

a) en faisant réagir l'hydrazide de formule

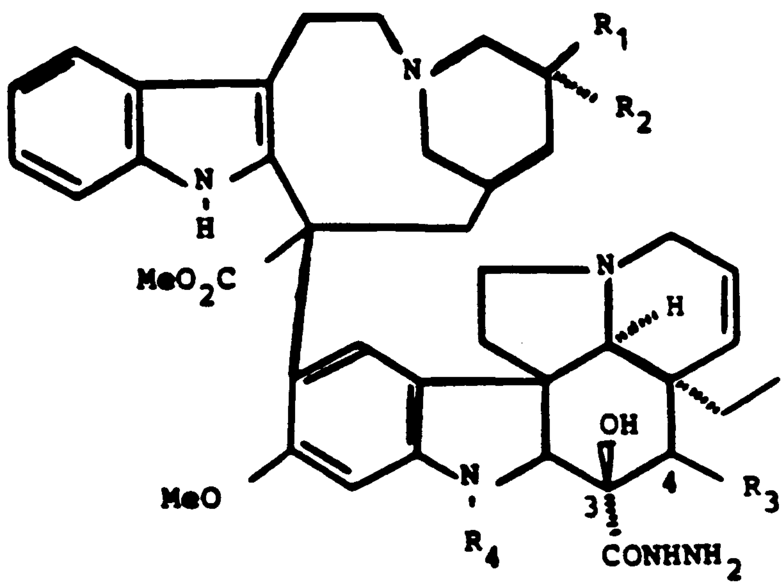

avec un nitrite pour former l'azide correspondant en position C-3 et

b) en faisant réagir l'azide avec une amine de formule :

H - A - R - COZ

la fonction COOH terminale de R étant protégée sous forme d'un groupe de protections connu dans la chimie des protéines Z, notamment le groupe benzyl,

c) en déprotégeant la fonction COOH terminale de R du dérivé obtenu après l'étape b).

2) on couple le dérivé obtenu à l'étape c) avec le transporteur polypeptidique

a) en activant la fonction COOH du dérivé obtenu à l'étape 1c) sous forme -COZ' dans laquelle Z'est un groupe labile connu dans la chimie des peptides qui permet

b) de réaliser un lien amide covalent avec un groupe amino libre du transporteur polypeptidique.

Z' peut être un groupe connu dans l'état de la technique tel que ceux décrits dans Peptide Synthesis de Bodanszky, Y.S. Klausner et M.A. Ondetti, Seconde Edition (1976) John Wiley & Sons, notamment pages 85 à 136. On peut citer pour Z' le groupe ($-N_3$), un halogène, notamment brome

ou chlore, un groupe acyloxy de formule $R^5CO.O$ dans laquelle $R^5$ est un reste aliphatique ou aromatique tel que, par exemple, un alkyl en $C_1$-$C_4$, un groupe alcoxy de préférence en $C_1$-$C_3$ ou un groupe aryloxy, un méthanesulphonyloxy, tosyloxy ou benzènesulphonyloxy, un radical imidazolyl ou le reste d'un dérivé N-acylhydroxylamine, par exemple Z' peut être un succinimidoxy, un phthalimidoxy ou un benzotriazolyloxy. Les exemples préférés sont les groupes N-acylhydroxylamine, notamment les esters N-hydroxysuccinimide préparés en utilisant le 1-cyclohexyl-3-(2-morpholinoéthyl)-carbodiimide méthop-toluènesulphonate ou 1,3 dicyclohexyl-carbodiimide ou via un anhydride mixte tel que ceux obtenus en utilisant l'isobutylchloroformate.

Quand Z′est un radical imidazolyl, il peut être préparé en utilisant un radical carbonyl di-imidazole et quand Z′ est $R^5CO.O$ avec $R^5$ qui est un alkyl $C_1$-$C_4$, notamment éthyl et isobutyl, il peut être préparé par utilisation d'un 1-éthoxycarbonyl-2-éthoxy-1,2-dihydroquinoline et l'isobutyl chloroformate respectivement.

La réaction est effectuée comme suit. L'hydrazide est dissous dans un solvant tel que le MeOH en milieu acide, HCl 1 N par exemple, la solution est refroidie et on ajoute un nitrite tel que le nitrite de sodium, le pH est alors ajusté avec une base faible telle que l'hydrogénocarbonate de sodium, on obtient ainsi un azide acide qui est extrait par un solvant non miscible tel que le dichlorométhylène.

Après séparation, l'azide, séché et concentré, est traité par l'amine correspondant au produit désiré (H-A-R-COZ) et le produit brut est séparé du mélange réactionnel et éventuellement purifié.

L'amide obtenu est séparé du milieu réactionnel par tout procédé approprié.

Le dérivé à fonction COO protégée est ensuite déprotégé,par exemple il est mis à hydrogèner dans du méthanol en présence de charbon palladié 10 %.

Enfin le couplage avec la protéine est de préférence effectué en milieu aqueux à une température entre 5°C et 25°C par exemple à la température ambiante et à pH entre 7,5 et 9,5, de préférence 8 et 9. Le couplage a lieu sur un groupe amino libre de la protéine vectrice, par exemple un groupe amino d'un reste lysine.

Enfin, l'invention a également pour objet, à titre de produit intermédiaire utile dans le procédé selon l'invention, un dérivé de vinca en C-3 comportant en position C-3 une chaîne détergente d'au moins 7 atomes de carbone aliphatique, chaîne substituée à son extrémité par le groupe COZ′, de l'étape 2a) du procédé.

L'invention concerne également l'application des conjugués à titre de médicament, ainsi que les compositions pharmaceutiques les incorporant et comportant des excipients, supports ou vecteurs pharmaceutiques acceptables.

Les composés selon la présente invention présentent en effet des propriétés antinéoplastiques qui les rendent particulièrement intéressantes dans le traitement des néoplasies malignes telles que les leucémies, la maladie de Hodgkin généralisée, certains lymphomes, les sarcomes ou carcinomes notamment.

D'autres caractéristiques et avantages de la présente invention apparaîtront à la lumière des exemples suivants illustrés par les figures 1 à 3 dans lesquels

. la figure 1 représente la masse tumorale relative en fonction du temps pour les hépatomes $HepG_2$ traités par le conjugué 2060 AHgal à différentes doses ;
. la figure 2 représente la courbe de stabilité dans le sérum d'un conjugué 2060 AHgal selon l'invention ;
. la figure 3 représente la courbel de digestion du même conjugué par les enzymes lysosomiaux.

EXEMPLE 1

Préparation de carboxamide de 4-désacétyl-vinblastine C-3 N-(11 carboxyundécyl) (composé n° 2060)

Le procédé est décrit en référence au schéma de synthèse I ci-aprés.

A) Carboxamide de 4-désacétyl-vinblastine C-3 N-(11 carbobenzyloxyundécyl) (3)

On dissout l'hydrazide de désacétyl-vinblastine (1) dont la préparation a été décrite dans (CONRAD et al. J. Med. Chem. 22,391 (1979)) à raison de 0,600 g (0.78 millimole) dans un mélange contenant 12 ml de méthanol et 36 ml de HCl 1N. La solution est refrodie à 0°C, puis est additionnée de 0,123 gr (1,78 millimoles) de $NaNO_2$ sec en une fois et sous agitation. Après 13 minutes, le pH de la solution est ajusté à 8,5 avec une solution saturée froide de $NaHCO_3$.

On extrait rapidement l'azide acide de la désacétyl-vinblastine avec 4 x 15 ml de $CH_2Cl_2$, puis on le lave avec une solution saturée de NaCl.

Les extraits sont séchés sur $Na_2SO_4$ et concentrés en un volume de 20 ml. A la solution de $CH_2Cl_2$, on ajoute du benzyl ester de l'acide 12-aminododécanoïque (2) à raison de 0,354 g (1,16 millimoles) et la solution est agitée pendant 2 heures à la température ambiante. La chromatographie en couche mince révèle un composant majeur (RF = O.46 ; méthanol : chlorure de méthylène 5:95).

Le solvant est évaporé et le résidu chromatographié sur silice en éluant avec un mélange d'acétate d'éthyle : méthanol (96:4). Les fractions appropriées sont combinées et les solvants enlevés pour conduire, à 450 mg de carboxamide de 4-désacétyl-vinblastine C-3 N-(11 carbobenzyloxyundécyl) (3) (rendement : 55 %), avec les caractéristiques physiques suivantes

RF : O.46 (silice, $CH_2Cl_2$ : $CH_3OH$ 95:5)

SM : Spectroscopie de masse (DCI, isobutane) 1043 ($M^+ + 1$), 1057 ($M^+ + 14 + 1$), 1025, 1011, 999, 985, 966, 951

RMN : ($CDCl_3$, 200 MHz) : 9,52 (OH, 1H), 8,03 (NH, 1H), 7,51 (H,H4, H-12′) (2H), 7,45-7,04 (Aromatiques, 7H), 6,61 (H-12, 1H), 6,07 (H-9, 1H), 5,83 (H-14, H-15, 2H), 5,12 ($CH_2$-Ph, 2H), 4,19 (H-17, 1H), 3,96 (H-17 A′, 1H), 3,77 ($OCH_3$, 3H), 3,58 (OMe, 3H), 3,35 (H-2), 2,79 (N-$CH_3$), 2,64 (H-21)

IR : (KBr, $cm^{-1}$): 3460, 2930, 2858, 1734, 1660, 1614, 1508, 1460, 1228, 740

Formule moléculaire : $C_{62}H_{83}N_5O_9$

Poids moléculaire : 1041,617.

B) carboxamide de 4-désacétyl-vinblastine C-3 N-(11 carboxyundécyl) (4)

Une solution de 400 mg (0,38 millimole) de 4-désacétyl-vinblastine C-3 N-(11 carbobenzyloxyundecyl) (3) dans 25 ml de méthanol est mise à hydrogéner à température ambiante et à pression atmosphérique en présence d'une pointe de spatule de Charbon Palladié 10 %. Après 15 heures d'agitation, 2 ml de méthanol saturé en $NH_3$ sont ajoutés et la solution est filtrée sur décalite®. Le filtre est lavé avec 2 x 10 ml de méthanol.

Le filtrat est concentré sous vide. Le résidu obtenu est ensuite purifié par chromatographie sur silice en utilisant un mélange d'éluants composé d'éther, de méthanol et d'ammoniaque aqueux 15 N. dans un rapport 50/49,5/0,5.

Les fractions appropriées sont combinées et les solvants enlevés pour conduire à 307 mg (0,32 millimole) de carboxamide de 4-désacétyl-vinblastine C-3 N-(11-carboxyundécyl). rdt : 84 %.

Après trituration dans un mélange d'acétate d'éthyle et d'éther de pétrole, le produit est obtenu sous forme d'une poudre blanche amorphe ayant les caractéristiques physiques suivantes :

RF : 0.33 (silice, $CH_2Cl_2$ : $CH_3OH$ 90:10)

SM : (DCI) 952, 892, 809, 749, 709, 663, 651, 610, 598, 571

RMN : ($CDCl_3$, 200 MHz) : 8,02 (NH, 1H), 7,52 (H-11, H-12, 2H), 6,49 (H-12, 1H), 6,07 (H-9, 1H), 5,81 (H-14-15, 2H), 4,15 (H-17, 1H), 3,77 (OMe, 3H), 3,58 (OMe, 3H), 3,33 (H-2), 2,79 (N-Me), 2,58 (H-21)

IR : ($KB_R$, $cm^{-1}$) 3460, 2925, 2853, 1719, 1660, 1613, 1501, 1460, 1229.

Formule moléculaire : $C_{55}H_{77}N_5O_9$

Poids moléculaire : 951,57

Le sel bis méthane sulfonate est préparé en dissolvant du carboxamide de 4-désacétyl-vinblastine C-3 (11 carboxyundécyl) (4) (110 mg) dans de l'éthanol anhydre et en ajoutant de l'acide éthanolique méthanesulfonique à 2 %. La solution est concentrée sous vide. On ajoute au résidu 3 ml d'eau distillée et la solution obtenue est lyophilisée pour donner 133 mg de sel bis-méthane sulfonate.

SCHEMA DE SYNTHESE I

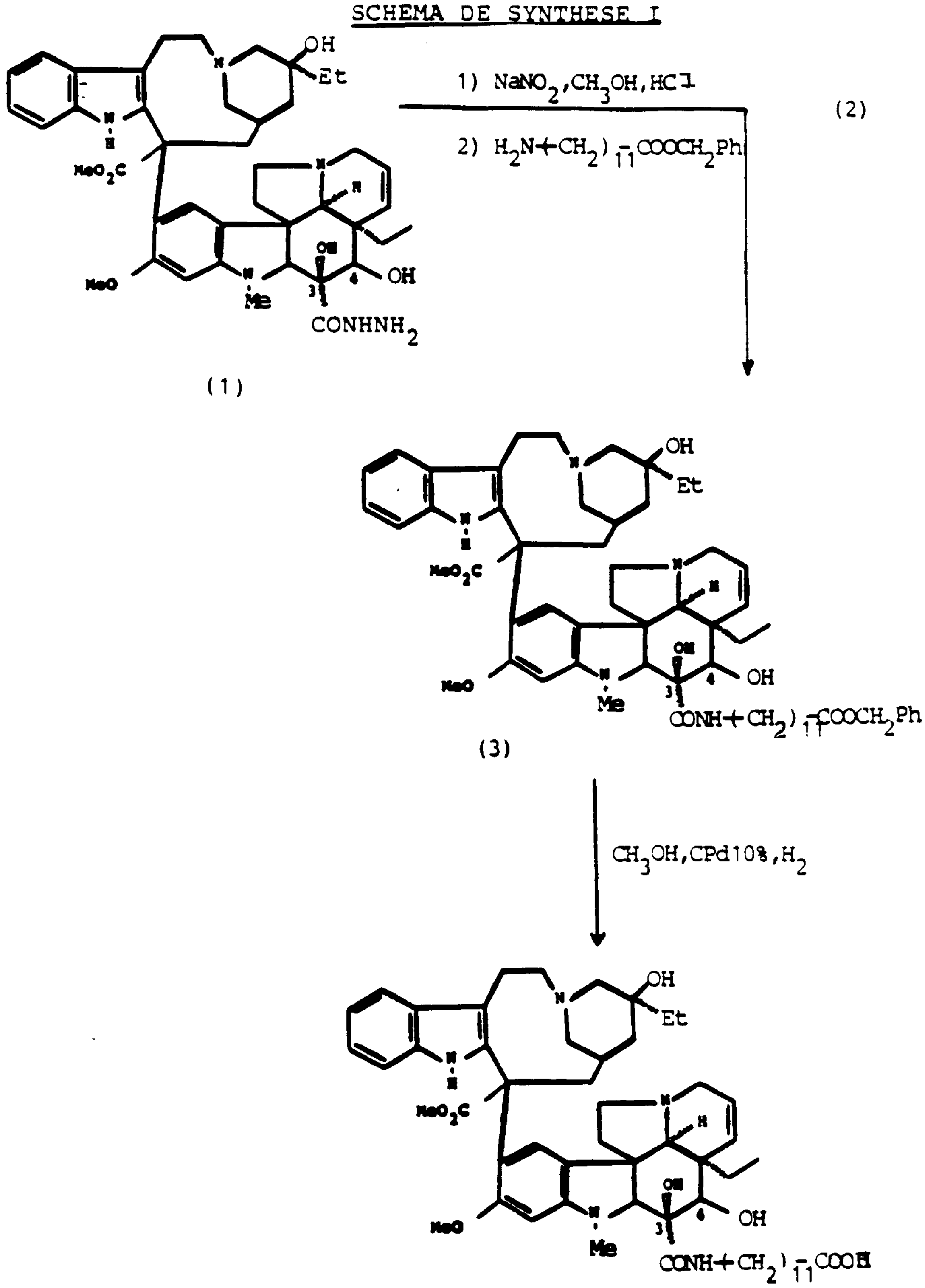


(1)

(3)

Composé 2060 (4)

EXEMPLE 2

Conjugué entre la carboxamide de 4-désacétyl-vinblastine C-3 N-(11 carboxyundécyl) (4) et l'albumine de sérum humain galactosylée

Une solution d'isobutylchloroformate (16 µl) dans le dioxanne sec (2mg) est ajoutée à 10 °C goutte à goutte sous agitation à de la carboxamide de 4-désacétylvinblastine C-3 N-(11 carboxyundécyl) (97 mg) et de la triéthylamine (17 µl) dissoutes dans le dioxanne (80 ml). Après 15 minutes d'agitation, le mélange organique est transféré goutte à goutte sous agitation dans 185 ml d'une solution d'albumine de sérum humain galactosylée (5,4 mg/ml) dans du tampon phosphate 0,15 M (pH 8,5).

Le mélange est agité à température ambiante pendant 15 heures. Il est ensuite purifié par filtration sur gel G25® équilibré avec une solution NaCl 9 ‰.

Le pic exclu est récupéré, filtré sur Millipak 40® (0,22 µ), concentré jusqu'à obtention d'un volume de 16 ml, et stérilisé.

Le contenu en protéines est mesuré par la technique de Lowry et le contenu en alcaloïdes estimé par mesure de la radioactivité.

Le conjugué ainsi préparé contient 6,2 moles de carboxamide de 4-désacétyl-vinblastine C-3 N-(11 carboxyundécyl) par mole d'albumine galactosylée.

- Mise au point de la technique de couplage du détergent 2060 ($V_3-(CH_2)_{11}-COOH$) à l'AHgal .

Il convient d'obtenir des conjugués ayant le rapport molaire médicament/transporteur maximal tout en gardant le meilleur rendement possible en forme monomère du transporteur macromoléculaire.

Au-delà d'un certain seuil dans le rapport molaire drogue/transporteur, il se produit très rapidement une polymérisation du transporteur le rendant inutilisable. Il apparaît que cette polymérisation affecte les propriétés de reconnaissance de la protéine.

| Rapport engagé moles vinca/moles lysine | % de dioxanne | Rapport obtenu moles vinca/moles AHgal | HPLC | | |
|---|---|---|---|---|---|
| | | | % Mono $7.10^4$ | % P.M. $15.10^4$ | % P.M. $67.10^4$ |
| 0,3 | 7,3 | 2 | 55 | 9 | 36 |
| 0,3 | 15 | 2,8 | 65 | 28 | 7 |
| 0,3 | 30 | 8 | 45 | 55 | - |
| 0,6 | 30 | 14,7 | 29 | 70 | 1 |
| 0,9 | 30 | 25 | 17 | 82 | 1 |

EXEMPLE 3

3.1 Activité chimiothérapeutique sur la leucémie P 388

Des essais d'activité antitumorale de dérivés de conjugués de la VLB des exemples 1 et 2 ont été conduits sur des souris de la façon suivante :

3.1.1. Paramètres d'activité

Deux paramètres d'activité sont utilisés :

- le paramètre "I.L.S." représente l'augmentation de la durée de vie, en pourcentage et selon la relation suivante :

I.L.S. % = $(\frac{T}{C} \times 100) - 100$

T (en jours) étant la durée moyenne de vie des souris traitées,
C (en jours) étant la durée moyenne de vie des souris témoins.

- le paramètre "survivants à long terme" dont le calcul en pourcentage est effectué au jour 6O.

3.1.2. Administration intrapéritonéale

$10^6$ cellules de leucémie $P_{388}$ sont inoculées par voie intrapéritonéale chez des souris $BDF_1$ femelles au jour O. Le lendemain, le composé 2060 est administré par voie intrapéritonéale.

| Produit | Dose mg/kg/jour | Nombre de souris | ILS % | Survivants au jour 60 | Remarques |
|---|---|---|---|---|---|
| 2060 | 25 | 7 | 82 | 0/7 | NaCl 9 ‰ |
| | 45 | 7 | 186 | 0/7 | $H_2O$ |
| | 50 | 7 | 105 | 0/7 | NaCl 9 ‰ |
| | 50 | 7 | 156 | 2/7 | $H_2O$ |
| | 75 | 7 | -38 | 0/7 | NaCl 9 ‰ |

- 2060 = 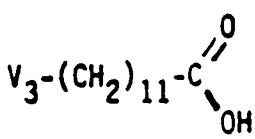

Le conjugué du dérivé 2060 couplé à l'albumine galactosylée humaine (AHg) a donné les résultats remarquables suivants.

| Activité chimiothérapeutique du conjugué 2060-AHg sur la leucémie P388 implantée par voie i.p. chez la souris $BDF_1$ femelle ($10^6$ cellules i.p., traitement i.p. au jour 1). | | | | | | | | |
|---|---|---|---|---|---|---|---|---|
| N° Exp. | Dose Vinca mg/kg | Dose Prot mg/kg | Nombre de souris | % ILS | Survivants au jour 60 | Rapport molaire | Lot conjugué | % Mono |
| 537 | 20 | 259 | 7 | >582 | 4/7 | 6,2 | 1166 | 59 |
| 543 | 20 | 318 | 7 | >689 | 4/7 | 5,1 | 1182 | 53 |
| 543 | 30 | 477 | 7 | 197 | 3/7 | 5,1 | 1182 | 53 |
| 537 | 40 | 517 | 7 | -31 | 0/7 | 6,2 | 1166 | 59 |
| 556 | 20 | 360 | 7 | 267 | 2/7 | 4,5 | 1205 | 57 |

3.2 - Activité chimiothérapeutique sur l'hépatome HepG2

Des fragments de l'hépatome HepG2 de plus ou moins 2 $mm^3$ sont implantés en sous-cutané bilatéral dorsal chez la souris nue Balb/c femelle au jour O. Lorsque les tumeurs atteignent une masse donnée (environ 500 mg), le traitement est administré par voie i.v. L'évolution des masses tumorales est suivie au cours du temps. Les diamètres tumoraux sont mesurés afin de déterminer la masse tumorale par la relation :

$$\text{Masse (mg)} = \frac{L\ (mm) \times l^2\ (mm)}{2} \quad \text{(au jour x)}$$

L = longueur ; l = largeur.

Les rapports des masses tumorales au jour x/masse tumorale au 1er jour du traitement sont portés en graphique en fonction du temps (masse relative ou MR/T).

Les produits administrés sont du NaCl 9 ‰ pour les contrôles et le conjugué 2060-AHgal aux doses respectives de 15, 20 et 25 mg/kg. Le graphique de la figure 1 reprend les moyennes des masses relatives en fonction du temps. Le traitement a été administré en 5 injections i.v. respectivement aux jours 26, 33, 40, 47 et 54.

Les résultats de la figure 1 montrent une inhibition partielle de croissance des tumeurs significatives aux trois doses évaluées.

Pour les HepG2, on observe une disparité très grande dans les tumeurs et il arrive qu'une tumeur d'une certaine masse arrête à un certain moment de se développer. C'est ce que l'on observe avec la série contrôle. En effet, la moyenne des masses relatives des contrôles évolue jusqu'à atteindre 25 fois la valeur de départ. Les souris avec les tumeurs les plus grosses meurent et on observe donc une chute de la moyenne. Celle-ci retombe à une valeur très faible correspondant à un hépatome qui a arrêté son développement. En effet, normalement la moyenne devrait retomber à 0.

En ce qui concerne l'effet du conjugué, on observe une inhibition très nette du développement des masses tumorales. Après l'arrêt du traitement, on observe une recrudescence des tumeurs. La survie des animaux traités est également supérieure à celle des contrôles.

## EXEMPLE 4

### 4.1 - Stabilité du conjugué 2060-AHgal dans le sérum

Le conjugué 2060-AHgal a été incubé à 37°C en présence de 80 % de sérum humain ou de NaCl 9 ‰ (sérum physiologique).

Après différents temps T d'incubation, les protéines non dégradées sont précipitées, après addition de 5 mg d'albumine sérique, par deux volumes d'acétonitrile.

Après incubation des échantillons à 4°C pendant une heure, ceux-ci sont centrifugés et la radioactivité du surnageant est estimée par comptage d'une partie aliquote en scintillation liquide.

La radioactivité soluble est une mesure de la digestion du conjugué.

Les valeurs figurant dans le tableau I ci-après représentent le pourcentage de radioactivité soluble (Vinca tritié).

Les résultats du tableau I reproduits également dans la figure 2 établissent la bonne stabilité du conjugué dans le sérum.

### 4.2 - Digestion du conjugué 2060-AHgal par les enzymes lysosomiales

Le conjugué a été incubé à 37°C en présence de cystéine 5 mM, de tampon acétate 40 mM et d'enzymes lysosomiales (T.S.) ou d'eau.

Après différentes temps T d'incubation, les protéines non dégradées sont précipitées, après addition de 5 mg d'albumine sérique, par deux volumes d'acétonitrile.

Après incubation des échantillons à 4°C pendant 40 minutes et centrifugation à 3000 RPM pendant 40 minutes, la radioactivité du surnageant est estimée par comptage d'une partie aliquote en scintillation liquide. La radioactivité soluble est une mesure de la digestion du conjugué.

Le pourcentage de radioactivité soluble reproduit dans le tableau I et dans la figure 3 montre qu'environ 80 % des conjugués ont été digérés par les enzymes lysosomiales (T.S.).

Tableau I

| $V_3$-$C_{12}$-AHgal (2060-AG) | | | | |
|---|---|---|---|---|
| STABILITE 80% SERUM HUMAIN | | | DIGESTION TS | |
| TEMPS(H) | NACL 9 ‰ | SERUM | $H_2O$ | TS |
| 0 | 14 | 12.2 | 12.3 | 14.3 |
| 24.3 | 16.8 | 8 | 11.2 | 67.8 |
| 47.55 | 16.6 | 9 | 12.3 | 77.3 |
| 71.5 | 13.3 | 18.4 | 14.1 | 82.8 |

EXEMPLE 5 - Couplage du carboxamide de 4-désacétyl-vinblastine C-3 N-(11-carboxyundécyl) à l'anticorps monoclonal (type IgG CTMO1)

Une solution d'isobutylchloroformate (2,8 µl) dans du dioxanne sec (0,5 ml) est ajoutée à 10 °C goutte à goutte sous agitation à de la carboxamide de 4-désacétyl-vinblastine C-3 N-(11 carboxyundécyl) (16 mg) et de la triéthylamine (2,6 µl) dissoutes dans 1 ml de dioxanne. Après 15 minutes d'agitation, le mélange organique est transféré goutte à goutte sous agitation dans 20 ml d'une solution d'anticorps monoclonal (8,5 mg P/ml) dans du tampon phosphate 0,1 M pH 8,5.

Le mélange est agité à température ambiante pendant 1 nuit. Il est ensuite purifié par filtration sur gel Sephadex G25® équilibré dans une solution de NaCl 9 ‰ pH 7,5. Le pic exclu est collecté, concentré par ultrafiltration et stérilisé.

Le contenu en protéines est mesuré par la méthode de Lowry et le contenu en alcaloïdes est estimé par mesure de la radioactivité.

Le conjugué ainsi préparé contient 1 mole de carboxamide de 4-désacétyl vinblastine C-3 N-(11 carboxyundecyl) par mole d'anticorps monoclonal.

## Revendications

**Revendications pour les Etats contractants suivants : AT, BE, CH, DE, FR, GB, IT, LI, LU, NL, SE**

**1.** Conjugués de dérivé de vinca (indoledihydroindole)-alcaloïde comportant en position C-3 une chaîne détergente d'au moins 7 atomes de carbone aliphatique, chaîne substituée à son extrémité par un radical COOH couplé à une fonction amino libre d'un transporteur macromoléculaire de nature polypeptidique par l'intermédiaire d'une liaison amide (-CONH-),
caractérisés en ce qu'ils répondent à la formule générale I

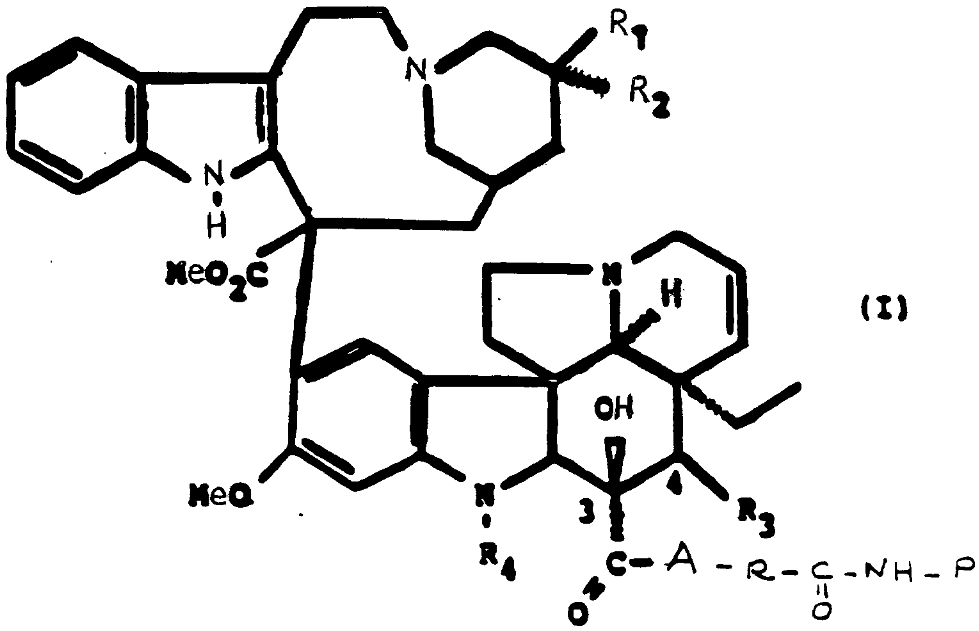

(I)

dans laquelle :

. $(R_1, R_2)$ représentent (-Et, -OH) ou (-H, Et)
. $R_4$ représente -H, -Me ou -CHO,
. $R_3$ représente -OH ou

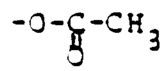

A étant -NH- ou le radical divalent d'un aminoacide entrant dans la constitution des protéines se terminant par -NH- et

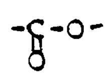

ou -NH- et

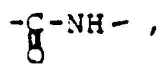

. R représente un radical hydrocarboné aliphatique en $C_7$ à $C_{20}$,
NH-P représente le reste du transporteur $NH_2P$ dans lequel $NH_2$ représente une fonction amino libre dudit transporteur.

**2.** Conjugués selon la revendication 1, caractérisés en ce que
. $R_3$ = OH
et
. A = -NH-.

**3.** Conjugués selon l'une des revendications 1 ou 2, caractérisés en ce que
. $(R_1, R_2)$ = (-Et, -OH) ,
et
. $R_4$ = Me.

**4.** Conjugés selon l'une des revendications 1 à 3, caractérisés en ce que
-A-R est choisi parmi-NH-$(CH_2)_{11}$ , -NH-$(CH_2)_{17}$ ,
-NH-$(CH_2)_7$-CH = CH-$(CH_2)_8$

**5.** Conjugués selon l'une des revendications précédentes, caractérisés en ce que le transporteur macromoléculaire est choisi parmi les protéines, néoglycoprotéines ou des fragments de celles-ci.

**6.** Conjugués selon la revendication 5, où le transporteur est choisi parmi :
. la sérum albumine de bovin
. l'albumine humaine galactosylée, ou
. les facteurs de croissance tels que l'EGF.

**7.** Conjugués selon l'une des revendications précédentes, caractérisés en ce que le transporteur est choisi parmi les immunoglobulines IgG, des fragments de celles-ci, telles que des anticorps monoclonaux.

**8.** Conjugué selon la revendication 7, caractérisé en ce que le transporteur est l'anticorps monoclonal CTM-01.

**9.** Procédé de préparation de conjugués selon l'une des revendications précédentes, caractérisé en ce que
1) on prépare le dérivé d'alcaloïde de vinca en C-3 à coupler
a) en faisant réagir l'hydrazide de formule

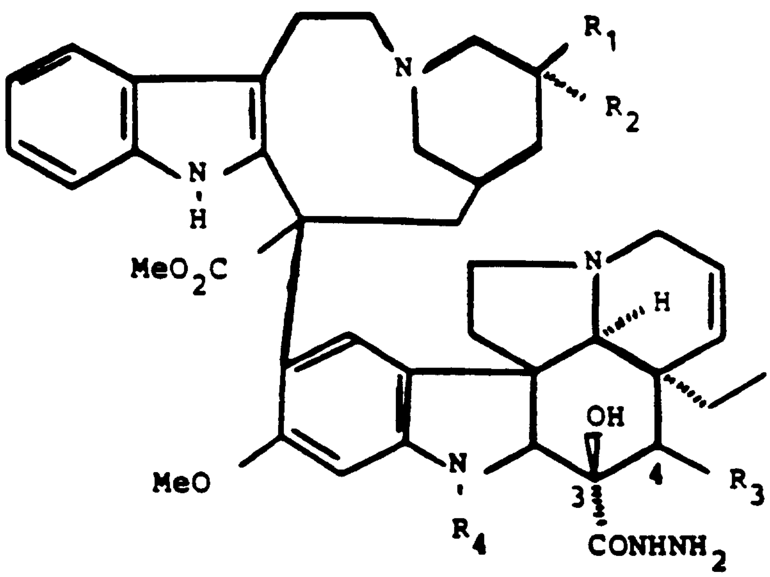

avec un nitrite pour former l'azide correspondant en position C-3 et
b) en faisant réagir l'azide avec une amine de formule :

H - A - R - COZ

la fonction COOH de R étant protégée sous forme d'un groupe de protections connu dans la chimie des protéines Z
c) en déprotégeant la fonction COOH de R du dérivé obtenu après l'étape b).
2) on couple le dérivé obtenu à l'étape c) avec le transporteur polypeptidique
a) en activant la fonction COOH du dérivé obtenu à l'étape 1c) sous forme -COZ' dans laquelle Z' est un groupe labile connu dans la chimie des peptides qui permet
b) de réaliser un lien amide covalent avec un groupe amino libre du transporteur polypeptidique.

**10.** Composition pharmaceutique comportant un conjugué selon l'une des revendications 1 à 8, à titre de principe actif.

**11.** Composition selon la revendication 10, caractérisée en ce qu'elle est sous forme injectable.

**Revendications pour les Etats contractants suivants : ES, GR**

**1.** Procédé de préparation d'un conjugué par couplage d'un dérivé de vinca (indole-dihydroindole)-alcaloide comportant en position C-3 une chaîne détergente d'au moins 7 atomes de carbone aliphatique, chaîne substituée à son extrémité par un radical COOH, à une fonction amino libre d'un transporteur macromoléculaire de nature polypeptidique par l'intermédiaire d'une liaison amide (-CONH-); ce conjugué répondant à la formule générale I

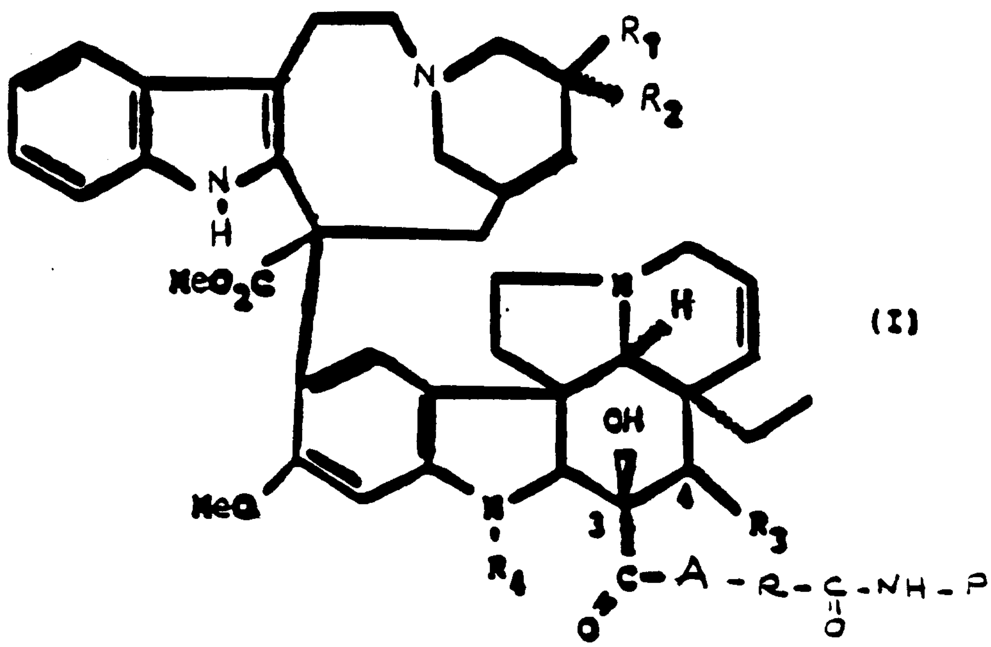

(I)

dans laquelle :

. ($R_1$, $R_2$) représentent (-Et, -OH) ou (-H, Et)
. $R_4$ représente -H, -Me ou -CHO,
. $R_3$ représente -OH ou

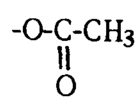

A étant -NH- ou le radical divalent d'un aminoacide entrant dans la constitution des protéines se terminant par -NH- et

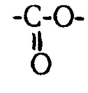

ou -NH- et

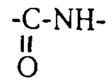

. R représente un radical hydrocarboné aliphatique en $C_7$ à $C_{20}$, NH-P représente le reste du transporteur $NH_2P$ dans lequel $NH_2$ représente une fonction amino libre dudit transporteur.

**2.** Procédé selon la revendication 1, caractérisé en ce que
. $R_3$ = OH,
et
. A = -NH-.

**3.** Procédé selon l'une des revendications 1 ou 2, caractérisé en ce que
. ($R_1$, $R_2$) = (-Et, -OH),
et
. $R_4$ = Me.

**4.** Procédé selon l'une des revendications 1 à 3, caractérisé en ce que -A-R est choisi parmi $-NH-(CH_2)_{11}$, $-NH-(CH_2)_{17}$, $-NH-(CH_2)_7-CH=CH-(CH_2)_8$

**5.** Procédé selon l'une des revendications précédentes, caractérisé en ce que le transporteur macromoléculaire est choisi parmi les protéines, néoglycoprotéines ou des fragments de celles-ci.

**6.** Procédé selon la revendication 5, où le transporteur est choisi parmi:
. la sérum-albumine de bovin,
. l'albumine humaine galactosylée, ou
. les facteurs de croissance tels que l'EGF.

**7.** Procédé selon l'une des revendications précédentes, caractérisé en ce que le transporteur est choisi parmi les immunoglobulines IgG, des fragments de celles-ci, telles que des anticorps monoclonaux.

**8.** Procédé selon la revendication 7, caractérisé en ce que le transporteur est l'anticorps monoclonal CTM-01.

**9.** Procédé de préparation de conjugués selon l'une des revendications précédentes, caractérisé en ce que
1) on prépare le dérivé d'alcaloïde de vinca en C-3 à coupler,
a) en faisant réagir l'hydrazide de formule

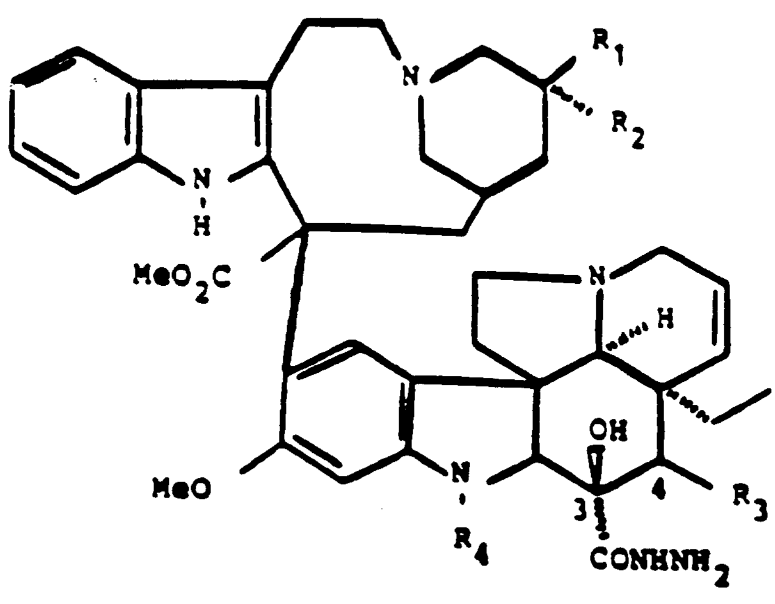

avec un nitrite pour former l'azide correspondant en position C-3, et

b) en faisant réagir l'azide avec une amine de formule:

H - A - R - COZ

la fonction COOH de R étant protégée sous forme d'un groupe de protections connu dans la chimie des protéines Z;

c) en déprotégeant la fonction COOH de R du dérivé obtenu après l'étape b).

2) on couple le dérivé obtenu à l'étape c) avec le transporteur polypeptidique

a) en activant la fonction COOH du dérivé obtenu à l'étape 1c) sous forme -COZ' dans laquelle Z' est un groupe labile connu dans la chimie des peptides qui permet

b) de réaliser un lien amide covalent avec un groupe amino libre du transporteur polypeptidique.

**Claims**

**Claims for the following Contracting States: AT, BE, CH, DE, FR, GB, IT, LI, LU, NL, SE**

**1.** Conjugates of a vinca (indole-dihydroindole) alkaloid derivative carrying a detergent chain of at least 7 aliphatic carbon atoms in the C-3 position, the said chain being substituted at its end by a COOH radical coupled to a free amino group of a macromolecular carrier of polypeptide character via an amide (-CONH-) bond, characterized in that they correspond to the general formula I

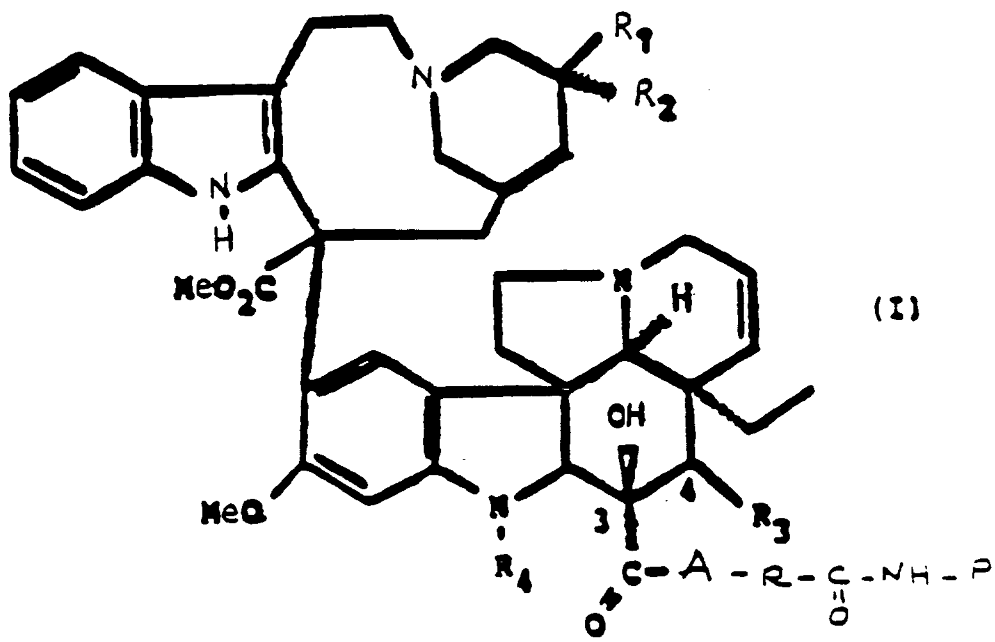

(I)

in which:

- ($R_1$, $R_2$) represent (-Et, -OH) or (-H, Et)
- $R_4$ represents -H, -Me or -CHO,
- $R_3$ represents -OH or

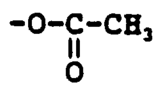

A being -NH- or the divalent radical of an amino acid entering into the structure of the proteins terminating in -NH- and

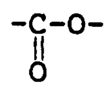

or -NH- and

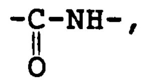

- R represents a $C_7$ to $C_{20}$ aliphatic hydrocarbon radical, NH-P represents the residue of the $NH_2P$ carrier in which $NH_2$ represents a free amino group of the said carrier.

**2.** Conjugates according to Claim 1, characterized in that
- $R_3$ = OH
  and
- A = -NH-.

**3.** Conjugates according to one of Claims 1 or 2, characterized in that
- ($R_1$, $R_2$) = (-Et, -OH),
  and
- $R_4$ = Me.

**4.** Conjugates according to one of Claims 1 to 3, characterized in that -A-R is chosen from $-NH-(CH_2)_{11}$, $-NH-(CH_2)_{17}$, $-NH-(CH_2)_7-CH=CH-(CH_2)_8$.

**5.** Conjugates according to one of the preceding claims, characterized in that the macromolecular carrier is chosen from proteins, neoglycoproteins or fragments of these.

**6.** Conjugates according to Claim 5, in which the carrier is chosen from:
- bovine albumin serum
- galactosylated human albumin or
- growth factors such as EGF.

**7.** Conjugates according to one of the preceding claims, characterized in that the carrier is chosen from IgG immunoglobulins and fragments of these, such as monoclonal antibodies.

**8.** Conjugate according to Claim 7, characterized in that the carrier is the CTM-01 monoclonal antibody.

**9.** Process for the preparation of conjugates according to one of the preceding claims, characterized in that
1) the C-3 vinca alkaloid derivative to be coupled is prepared

a) by reacting the hydrazide of formula

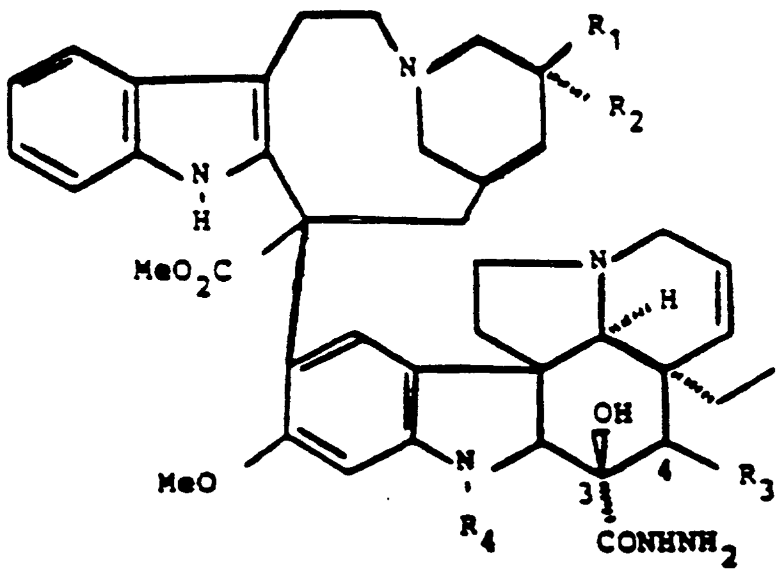

with a nitrite in order to form the corresponding azide in the C-3 position and
b) by reacting the azide with an amine of formula

H - A - R - COZ

the COOH group of R being protected in the form of a protective group known in Z protein chemistry, and
c) by removing the protection from the COOH group of R in the derivative obtained after step b).
2) the derivative obtained in step c) is coupled with the polypeptide carrier
a) by activating the COOH group in the derivative obtained in step 1c) in the form of -COZ', in which Z' is a labile group known in peptide chemistry which enables
b) a covalent amide bond to be produced with a free amino group of the polypeptide carrier.

**10.** Pharmaceutical composition containing a conjugate according to one of Claims 1 to 8 as the active ingredient.

**11.** Composition according to Claim 10, characterized in that it is in an injectable form.

**Claims for the following Contracting States: ES, GR**

**1.** Process for the preparation of a conjugate by coupling a vinca (indole-dihydroindole) alkaloid derivative carrying a detergent chain of at least 7 aliphatic carbon atoms in the C-3 position, the said chain being substituted at its end by a COOH radical, to a free amino group of a macromolecular carrier of polypeptide character via an amide (-CONH-) bond, this conjugate corresponding to the general formula I

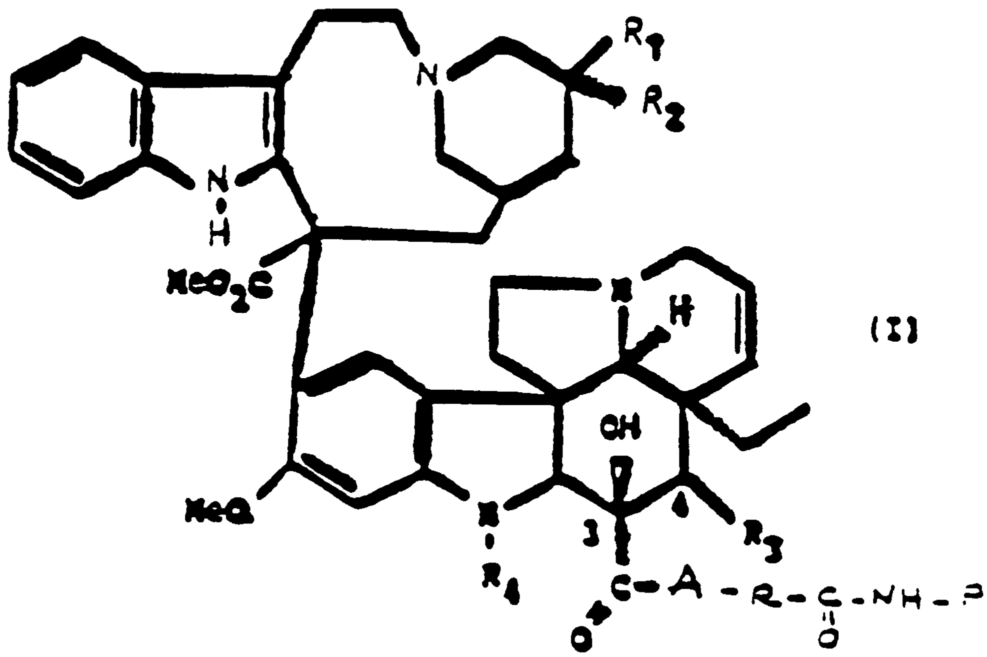

(I)

in which:

- ($R_1$, $R_2$) represent (-Et, -OH) or (-H, Et)
- $R_4$ represents -H, -Me or -CHO,
- $R_3$ represents -OH or

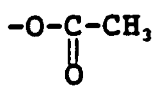

A being -NH- or the divalent radical of an amino acid entering into the structure of proteins terminating by -NH- and

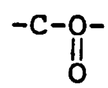

or -NH- and

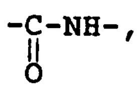

- R represents a $C_7$ to $C_{20}$ aliphatic hydrocarbon radical, NH-P represents the residue of the $NH_2P$ carrier in which $NH_2$ represents a free amino group of the said carrier.

**2.** Process according to Claim 1, characterized in that
- $R_3$ = OH, and
- A = -NH-.

**3.** Process according to one of Claims 1 or 2, characterized in that
- ($R_1$, $R_2$) = (-Et, -OH), and
- $R_4$ = Me.

**4.** Process according to one of Claims 1 to 3, characterized in that -A-R is chosen from $-NH-(CH_2)_{11}$, $-NH-(CH_2)_{17}$, $-NH-(CH_2)_7-CH=CH-(CH_2)_8$.

**5.** Process according to one of the preceding claims, characterized in that the macromolecular carrier is chosen from proteins, neoglycoproteins or fragments of these.

**6.** Process according to Claim 5, in which the carrier is chosen from:
- bovine albumin serum
- galactosylated human albumin or
- growth factors such as EGF.

**7.** Process according to one of the preceding claims, characterized in that the carrier is chosen from IgG immunoglobulins and fragments of these, such as monoclonal antibodies.

**8.** Process according to Claim 7, characterized in that the carrier is the CTM-01 monoclonal antibody.

**9.** Process for the preparation of conjugates according to one of the preceding claims, characterized in that
1) the C-3 vinca alkaloid derivative to be coupled is prepared
a) by reacting the hydrazide of formula

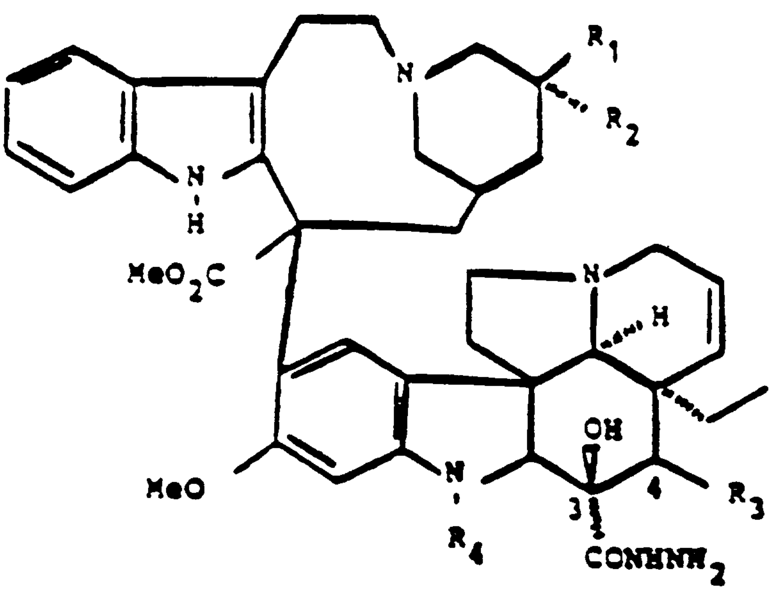

with a nitrite in order to form the corresponding azide in the C-3 position and
b) by reacting the azide with an amine of formula

H - A - R - COZ

the COOH group of R being protected in the form of a protective group known in Z protein chemistry, and
c) by removing the protection from the COOH group of R in the derivative obtained after step b).
2) the derivative obtained in step c) is coupled with the polypeptide carrier
a) by activating the COOH group in the derivative obtained in step 1c) in the form of -COZ', in which Z' is a labile group known in peptide chemistry which enables
b) a covalent amide bond to be produced with a free amino group of the polypeptide carrier.

## Patentansprüche

**Patentansprüche für folgende Vertragsstaaten : AT, BE, CH, DE, FR, GB, IT, LI, LU, NL, SE**

1. Konjugate eines Vinca(Indol-dihydroindol)-Alkaloid-Derivats, die in C-3-Position eine Detergens-Kette mit mindestens 7 aliphatischen Kohlenstoffatomen aufweisen, die an ihrem Ende durch einen COOH-Rest substituiert ist, der gekuppelt ist an eine freie Aminofunktion eines makromolekularen Transportmittels vom Polypeptid-Typ über eine Amid(-CONH-)-Bindung,
dadurch gekennzeichnet, daß sie die allgemeine Formel (I) haben

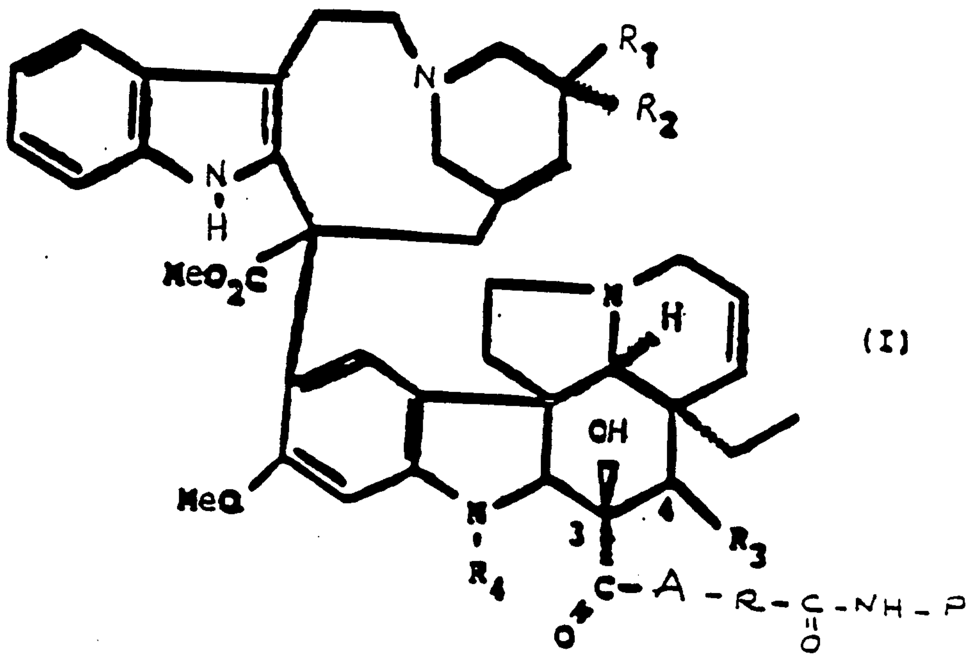

(I)

worin bedeuten:

. ($R_1$, $R_2$) (-Et, -OH) oder (-H, Et)
. $R_4$ -H, -Me oder -CHO,
. $R_3$ -OH oder

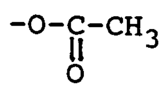

. A -NH- oder den divalenten Rest einer in die Struktur der Proteine eintretenden Aminosäure, der die Endgruppen -NH- und

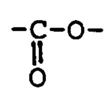

oder -NH- und

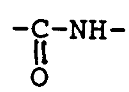

aufweist,
. R einen aliphatischen $C_7$-$C_{20}$-Kohlenwasserstoffrest und
. NH-P den Rest des Transportmittels $NH_2P$, in dem $NH_2$ eine freie Aminofunktion des Transportmittels darstellt.

2. Konjugate nach Anspruch 1, dadurch gekennzeichnet, daß
   . $R_3$ = OH
   und
   . A = -NH-.

3. Konjugate nach einem der Ansprüche 1 oder 2, dadurch gekennzeichnet, daß
   . ($R_1$, $R_2$) = (-Et, -OH)
   und
   . $R_4$ = Me.

4. Konjugate nach einem der Ansprüche 1 bis 3, dadurch gekennzeichnet, daß -A-R ausgewählt wird aus $-NH-(CH_2)_{11}$, $-NH-(CH_2)_{17}$ und $-NH-(CH_2)_7-CH=CH-(CH_2)_8$.

5. Konjugate nach einem der vorhergehenden Ansprüche, dadurch gekennzeichnet, daß das makromolekulare Transportmittel ausgewählt wird aus den Proteinen, Neoglycoproteinen oder Fragmenten derselben.

6. Konjugate nach Anspruch 5, in denen das Transportmittel ausgewählt wird aus
   . dem Rinderserumalbumin
   . dem galactosylierten Humanalbumin oder
   . den Wachstumsfaktoren, wie z.B. EGF.

7. Konjugate nach einem der vorhergehenden Ansprüche, dadurch gekennzeichnet, daß das Transportmittel ausgewählt wird aus den Immunglobulinen IgG und Fragmenten derselben, wie z.B. monoklonalen Antikörpern.

8. Konjugate nach Anspruch 7, dadurch gekennzeichnet, daß das Transportmittel der monoklonale Antikörper CTM-01 ist.

9. Verfahren zur Herstellung der Konjugate nach einem der vorhergehenden Ansprüche, dadurch gekennzeichnet, daß man
   1) das in der C-3-Position kuppelnde Vinca-Alkaloid-Derivat herstellt, indem man
   a) das Hydrazid der Formel

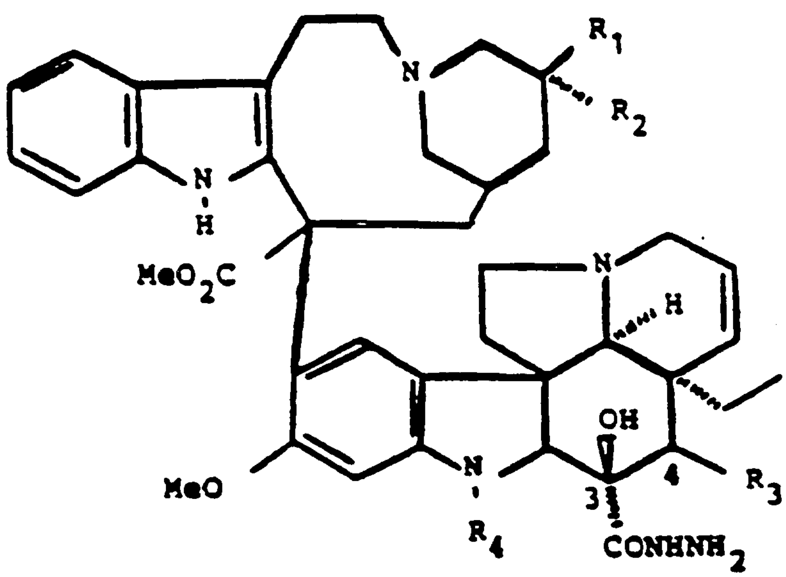

mit einem Nitrit reagieren läßt unter Bildung des entsprechenden Azids in der C-3-Position, und
b) das Azid mit einem Amin der Formel

H - A - R - COZ

reagieren läßt, in dem die COOH-Funktion von R in Form einer in der Chemie der Proteine bekannten Schutzgruppe Z geschützt ist,

c) wobei man die COOH-Funktion von R des nach der Stufe (b) erhaltenen Derivats von der Schutzgruppe befreit, und

2) das in der Stufe (c) erhaltene Derivat mit dem Polypeptid-Transportmittel kuppelt,

a) indem man die COOH-Funktion des in der Stufe (1c) erhaltenen Derivats in der -COZ'-Form, in der Z' eine in der Peptidchemie bekannte labile Gruppe darstellt, aktiviert, welche

b) die Ausbildung einer kovalenten Amidbindung mit einer freien Aminogruppe des Polypeptid-Transportmittels erlaubt.

**10.** Pharmazeutische Zusammensetzung, die ein Konjugat nach einem der Ansprüche 1 bis 8 als Wirkstoff (aktives Prinzip) enthält.

**11.** Zusammensetzung nach Anspruch 10, dadurch gekennzeichnet, daß sie in einer injizierbaren Form vorliegt.

**Patentansprüche für folgende Vertragsstaaten : ES, GR**

**1.** Verfahren zur Herstellung eines Konjugats durch Kuppeln eines Vinca(Indol-dihydroindol)-Alkaloid-Derivats, das in C-3-Position eine Detergens-Kette mit mindestens 7 aliphatischen Kohlenstoffatomen enthält, die an ihrem Ende durch einen COOH-Rest substituiert ist, an eine freie Aminofunktion eines makromolekularen Transportmittels vom Polypeptid-Typ über eine Amid(-CONH-)-Bindung, wobei dieses Konjugat die allgemeine Formel (I) hat:

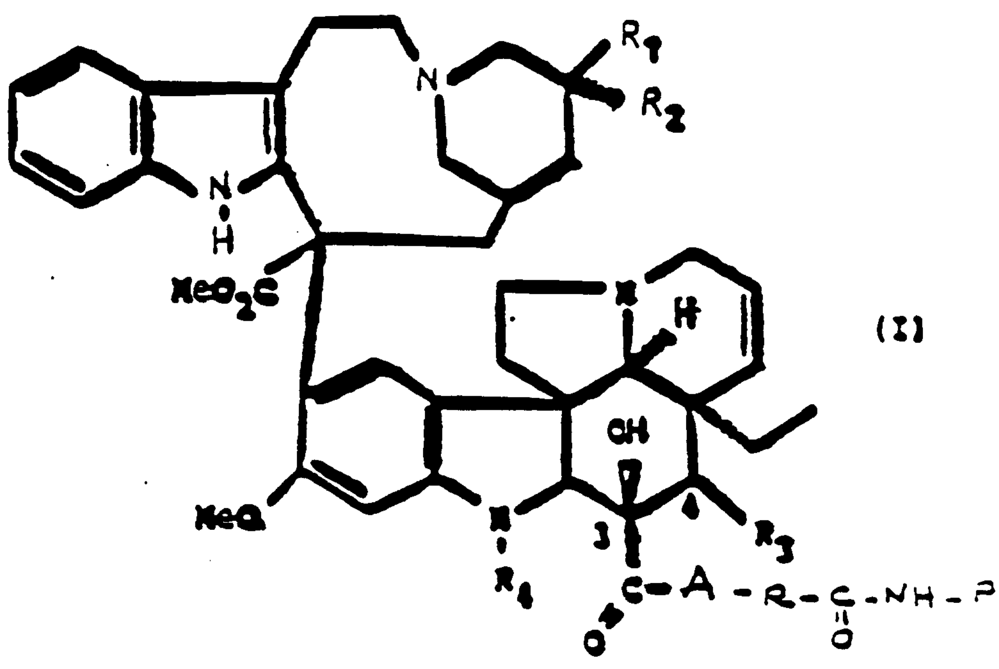

(I)

worin bedeuten:

. ($R_1$, $R_2$) (-Et, -OH) oder (-H, Et)

. $R_4$ -H, -Me oder -CHO,

. $R_3$ -OH oder

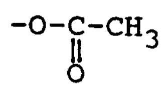

. A -NH- oder den divalenten Rest einer in die Struktur der Proteine eintretenden Aminosäure, der die Endgruppen -NH- und

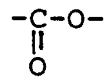

oder -NH- und

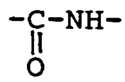

aufweist,

. R einen aliphatischen $C_7$-$C_{20}$-Kohlenwasserstoffrest und

. NH-P den Rest des Transportmittels $NH_2P$, in dem $NH_2$ eine freie Aminofunktion des Transportmittels darstellt.

**2.** Verfahren nach Anspruch 1, dadurch gekennzeichnet, daß

. $R_3$ = OH

und

. A = -NH-.

**3.** Verfahren nach einem der Ansprüche 1 oder 2, dadurch gekennzeichnet, daß

. ($R_1$, $R_2$) = (-Et, -OH)

und

. $R_4$ = Me.

**4.** Verfahren nach einem der Ansprüche 1 bis 3, dadurch gekennzeichnet, daß -A-R ausgewählt wird aus $-NH-(CH_2)_{11}$, $-NH-(CH_2)_{17}$ und $-NH-(CH_2)_7-CH=CH-(CH_2)_8$.

**5.** Verfahren nach einem der vorhergehenden Ansprüche, dadurch gekennzeichnet, daß das makromolekulare Transportmittel ausgewählt wird aus den Proteinen, Neoglycoproteinen oder Fragmenten derselben.

**6.** Verfahren nach Anspruch 5, bei dem das Transportmittel ausgewählt wird aus

. dem Rinderserumalbumin,

. dem galactosylierten Humanalbumin oder

. den Wachstumsfaktoren, wie z.B. EGF.

**7.** Verfahren nach einem der vorhergehenden Ansprüche, dadurch gekennzeichnet, daß das Transportmittel ausgewählt wird unter den Immunglobulinen IgG und Fragmenten derselben, wie z.B. monoklonalen Antikörpern.

**8.** Verfahren nach Anspruch 7, dadurch gekennzeichnet, daß es sich bei dem Transportmittel um den monoklonalen Antikörper CTM-01 handelt.

**9.** Verfahren zur Herstellung von Konjugaten nach einem der vorhergehenden Ansprüche, dadurch gekennzeichnet, daß man

1) das in der C-3-Position kuppelnde Vincaalkaloid-Derivat herstellt, indem man

a) das Hydrazid der Formel

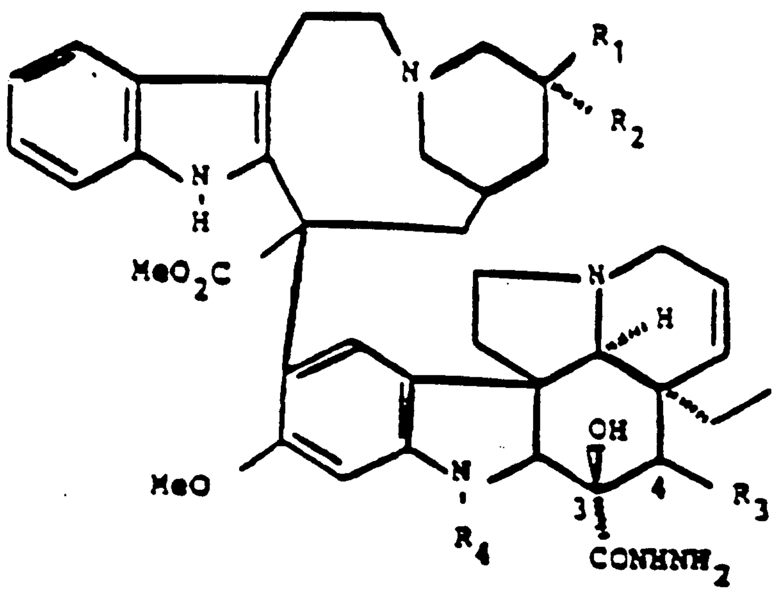

mit einem Nitrit reagieren läßt unter Bildung des entsprechenden Azids in der C-3-Position und
b) das Azid mit einem Amin der Formel

H - A - R - COZ

reagieren läßt, in dem die COOH-Funktion von R in Form einer in der Chemie der Proteine bekannten Schutzgruppe Z geschützt ist,
c) wobei man die COOH-Funktion von R des nach der Stufe (b) erhaltenen Derivats von der Schutzgruppe befreit, und
2) das in der Stufe (c) erhaltene Derivat mit dem Polypeptid-Transportmittel kuppelt,
a) indem man die COOH-Funktion des in der Stufe (1c) erhaltenen Derivats in der -COZ'-Form, in der Z' eine in der Peptidchemie bekannte labile Gruppe darstellt, aktiviert, welche
b) die Ausbildung einer kovalenten Amidbindung mit einer freien Aminogruppe des Polypeptid-Transportmittels erlaubt.

FIG_1

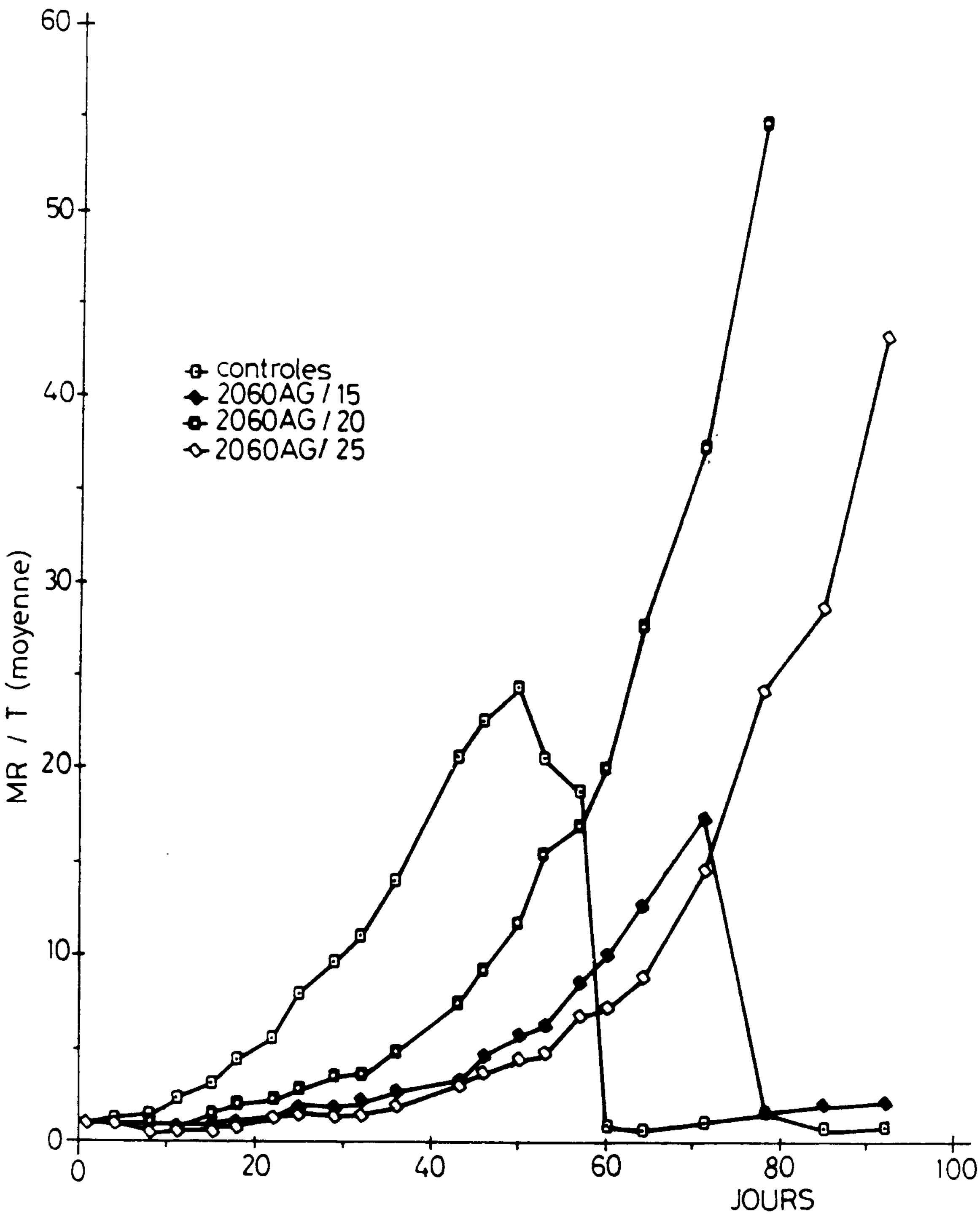
60
50
40
30
20
10
0
MR / T (moyenne)
controles
2060AG / 15
2060AG / 20
2060AG/ 25
0
20
40
60
80
100
JOURS

FIG_2

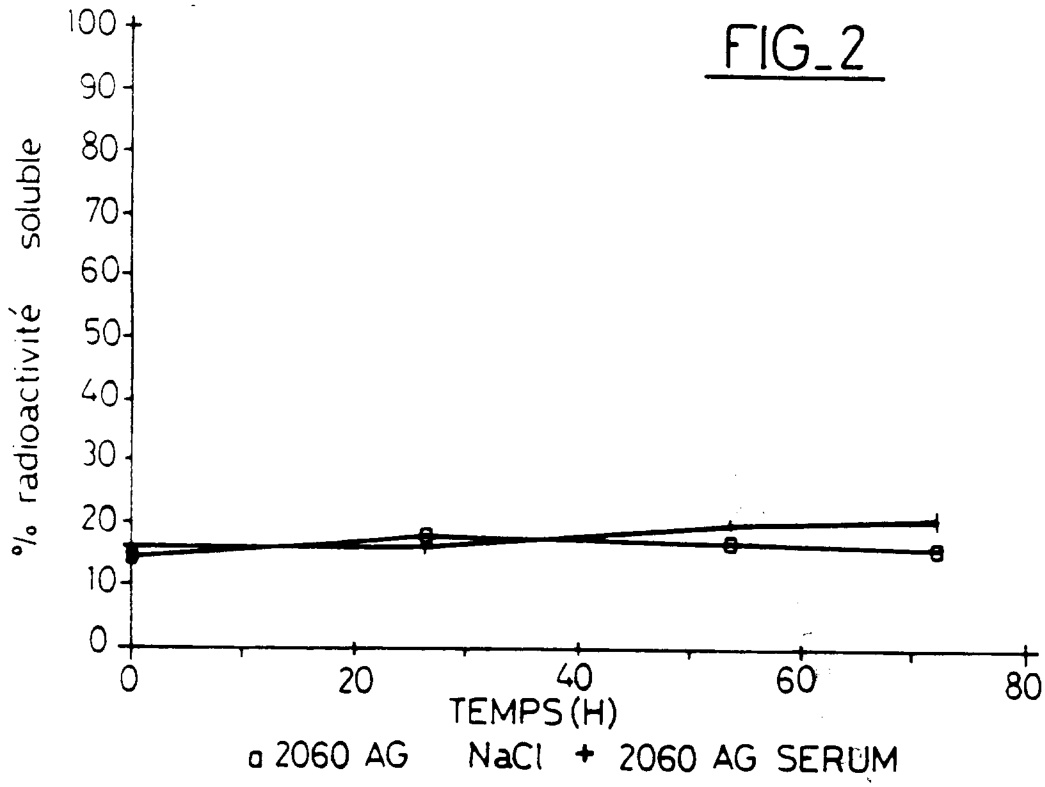
% radioactivité soluble
100
90
80
70
60
50
40
30
20
10
0
0
20
40
60
80
TEMPS (H)
▫ 2060 AG NaCl + 2060 AG SERUM

FIG_3

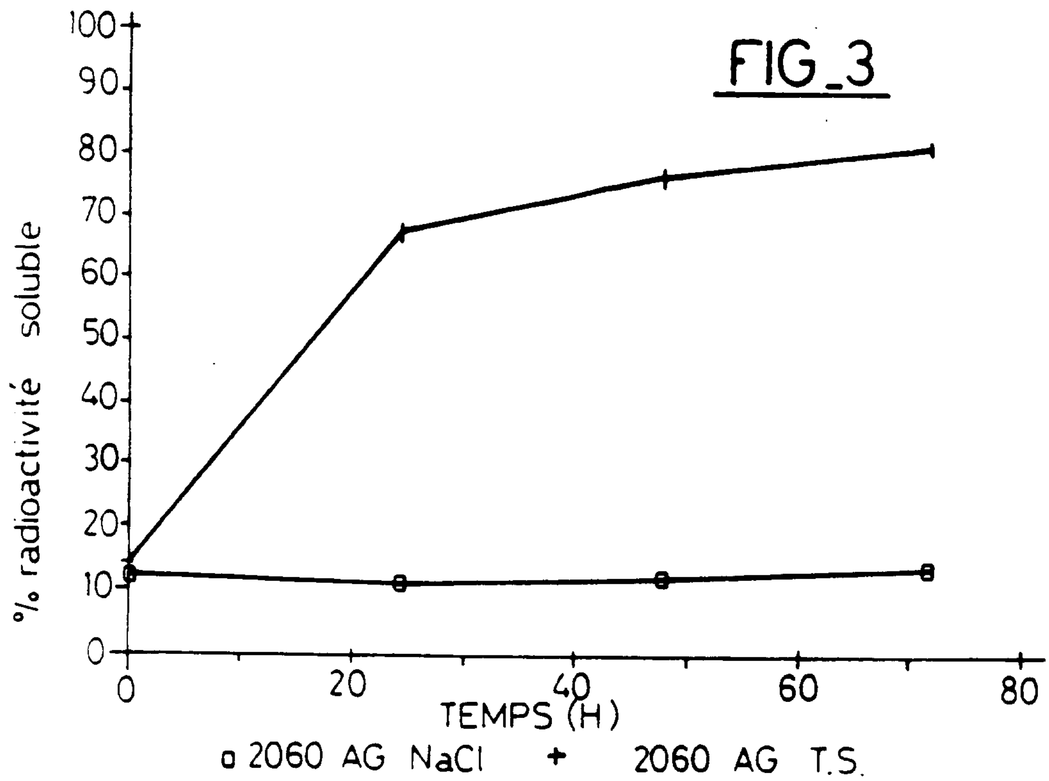
% radioactivité soluble
100
90
80
70
60
50
40
30
20
10
0
0
20
40
60
80
TEMPS (H)
▫ 2060 AG NaCl + 2060 AG T.S.